# EUROPEAN PATENT SPECIFICATION

(11) **EP 3 005 939 B1**
(45) Date of publication and mention of the grant of the patent: **13.12.2017**
(21) Application number: 15188329.5
(22) Date of filing: 05.10.2015
(51) Int. Cl.: A61B 5/00, A61B 5/20, E03D 9/00

(54) **TOILET UNIT**
TOILETTENEINHEIT
UNITÉ DE TOILETTE

(30) Priority: 06.10.2014 NL 2013582
(43) Date of publication of application: 13.04.2016
(73) Proprietor: Porta Holding B.V., 3911 TR Rhenen (NL)
(72) Inventor: Aufenacker, Robert Cornelis, 3911 TR Rhenen (NL); Groeneveld-Pors, Geertruida Anna, 3911 TR Rhenen (NL)
(74) Representative: Ellens, Andries

(56) References cited:
- DE-A1- 19 613 306
- GB-A- 2 437 549
- JP-A- H07 259 166

## Description

### FIELD OF THE INVENTION

The invention relates to a toilet arrangement for a urodynamic measurement. Further, the invention relates to a method of producing the toilet arrangement and a method of constructing the toilet arrangement (in an existing building).

### BACKGROUND OF THE INVENTION

Urine flow meters are known in the art. US51761488A, for instance, describes a device for measuring the urine flow of a patient, consisting of a measuring head provided with a funnel and an out-flow port, and of an electronic evaluating unit. The measuring head is provided with a slotted pilot tube forming the outflow port and with a pressure sensor measuring the dynamic pressure, and the electronic evaluating unit calculates the flow values from the flow-proportional dynamic pressure measured. GB2437549 discloses a toilet arrangement for a urodynamic measurement, the toilet arrangement comprising: a bowl unit with a bowl unit outlet; a weigh unit comprising: a receptor comprising (a) a receptor opening in fluid contact with the bowl unit outlet but not in direct physical contact with the bowl unit outlet and (b) a receptor outlet; a load cell configured to weigh the receptor and to provide a corresponding load cell signal; and a control unit configured to detect the load cell signal as function of time.

### SUMMARY OF THE INVENTION

The measurement of urine flow rate and volume is part of many standard urological examinations. Known prior art measuring devices are based on the principle of rotation-dynamic or volumetric or flow methods, wherein the patient's urine flow (over time) is determined by the rotation-dynamics of a disc or the volume collected during (complete) micturition. In the past years, urodynamics has developed into a sophisticated high-tech discipline. Electronically controlled measurements of complex functions provide reliable data for well-founded diagnoses. However, urine flow rates are still largely measured in the conventional way using more slowly evolved measuring devices. Using these device may result in measurement inaccuracy when patients, for instance, unintentionally knock against the flow meter, producing artefacts that falsify the reading.

Different kinds of chairs and stands in connection with urine flow meters have been introduced the last decades to comfort the patients and make them more at ease during the measurement and thus minimizing negative effects of the measurement conditions during micturition compared to "normal" micturition. Likewise, the implementation and analysis of simple and standard measurements have improved in the last years. Yet, fecal incontinence and urinary incontinence may both prevail, especially under elderly patients. Moreover, experience shows that patients accidentally also may excrete feces during the measurement, may use toilet paper, or unintentionally knock over a beaker used to collect the urine. Of coarse, this affects the measurement, but is also implicates extra cleaning for the person leading or assisting the measurement (e.g., a doctor, a nurse or an assistant, etc.).

Hence, it is an aspect of the invention to provide an alternative urine flow measurement system, which preferably further at least partly obviates one or more of above-described drawbacks.

In a first aspect, the invention provides a toilet arrangement for a urodynamic measurement, the toilet arrangement comprising (A) a bowl unit with a bowl unit outlet, (B) a weigh unit comprising (i) a receptor comprising (a) a receptor opening in fluid contact with the bowl unit outlet but not in direct physical contact with the bowl unit outlet and (b) a receptor outlet, (ii) a pump unit comprising (a) a pump unit inlet in fluid contact with the receptor outlet, (b) a shredder in fluid contact with the receptor outlet, and (c) a pump unit outlet, (iii) a load cell configured to weigh the receptor and the pump unit and to provide a corresponding load cell signal, and (C) a control unit configured to detect the load cell signal as function of time.

With such an arrangement the urine flow measurement may be performed easier and more hygienic, especially in case of any accidents. Additionally, it may improve the working conditions of the person leading or assisting the measurement. After the measurement, especially after having weighed all urine by the load cell, all material, especially urine, but possibly also feces and/or toilet paper, collected in the receptor can be removed via the receptor outlet by the pump unit to the pump unit outlet.

Urine (and other materials like feces, toilet paper and flushing fluid) from the bowl unit can flow from the bowl unit outlet to the receptor opening by gravity. Therefore, in a specific embodiment, the bowl unit and the receptor are configured providing the bowl unit outlet directly above the receptor opening, ensuring that all urine (and other material) from the bowl unit may enter the receptor, especially via the receptor inlet. The receptor may be substantially open at the top side, so all material can flow freely from the bowl unit outlet to the receptor opening.

The receptor opening also may be configured providing an opening in the top side of the receptor. If the receptor opening comprises an opening in the top side of the receptor, than, the cross-section of the receptor opening is especially at least be as large as the cross-section of the bowl unit outlet, their shapes are especially substantially the same and the bowl unit outlet should be provided substantially vertical over the receptor opening, to ensure that all material flowing out of the bowl unit outlet also will be received by the receptor. More preferably, the cross-section of the opening in the top side of the receptor is larger than the cross-section of the bowl unit outlet. For instance, the receptor opening may have the same diameter as or larger than the bowl unit opening.

In specific embodiments, the bowl unit outlet comprises a circular outlet, and the receptor inlet comprises a circular inlet, wherein the center of the bowl unit outlet is provided substantially vertical over the center of the receptor inlet. Especially, the ratio of the diameter of the receptor inlet to the diameter of the bowl unit outlet is at least 1, preferably 1.5, even more preferably the ratio is larger than 2. In another embodiment, the receptor has an open side at the top and the receptor opening comprises the entire cross-section of the top of the receptor.

The bowl unit may include a bowl and optionally other elements like a seat, a closure, etc. The bowl comprises an outlet, which may be identical to the bowl unit outlet.

In yet other embodiments, material guides, such as tubing, gutters, etc., may be provided to direct the urine (or other material) from the bowl unit outlet to the receptor inlet and the size of the cross-section of the receptor opening may be selected to be smaller, larger or having the same size as the size of the cross-section of the bowl unit outlet, preferably the size of the cross-section of the receptor opening is larger than the size of the cross-section of the bowl unit outlet. Such physical connection between the bowl unit outlet and receptor inlet especially comprises a flexible material. In this way, the receptor may move in a vertical direction independent from the bowl unit.

The bowl unit, however, is especially configured not to be in physical contact with the receptor opening. And the bowl unit - alone or, e.g., in combination with a patient sitting on the bowl unit - therefore, does not contribute to the weight measured by the load cell. The fact that the bowl unit and receptor (opening) are not in direct physical contact does not exclude the connecting part such as a flexible tube. The term "not in direct physical contact" especially indicates that the weight of the bowl unit has no influence on the weight measured by the load cell. Especially, there is a non zero distance between the receptor and the bowl unit. This non-zero distance may e.g. be in the range of 0.5-20 cm, such as 0.5-10 cm. This distance may be bridged by a flexible tube. However, the bowl unit and weight unit are not in direct contact with each other. Hence, though the bowl unit and the weight unit may be comprised in the same arrangement, the former has especially no influence on the signal measured by the weight unit (more precisely the load cell).

In a specific embodiment, the pump unit comprises a shredder, especially configured for shredding (semi-)solid material like feces and toilet paper, such as for preventing blockage downstream of the shredder.

The pump unit, further, comprises a pump to move the material. Different kind of pumps, such as a rotary vane pump, a gear pump, a lobe pump, a peristaltic pump, an impeller pump, a centrifugal pump, a progressive cavity pump, etc., may be selected for this function. The term pump may optionally also refer to a plurality of pumps. Depending on the type of pump, it may be advantageous to provide the shredder upstream of the pump. It also may be advantageous to provide the shredder downstream of the pump. It may also be advantageous to combine the pumping and the shredding action. Centrifugal pumps, for instance, can be provided with different types of impellers, ranging from propellers to slurry impellers and shredder impellers. Moreover, next to selecting one specific kind of impeller, combinations of different displacement and shredding principles may be combined in a pump. Hence in a specific embodiment the pump unit comprises a pump with an integrated shredder.

The bowl unit is configured to receive urine and to direct the urine via the bowl unit outlet to the receptor, especially to the receptor inlet. In an embodiment the bowl unit comprises a bowl shaped urine receiver ("bowl") configured to receive urine (and optionally faces, etc.) while a patient is standing up while micturiting. The bowl unit, further, may comprise a lid to close the urine receiver when no measurements are performed. In another embodiment the bowl unit comprises a toilet bowl ("bowl") with a toilet seat. In yet another embodiment the toilet seat is provided with a lid or closure that can be closed when the toilet arrangement is not in use or the lid can make the seat available again when a measurement is performed.

It further is advantageous that the holding time of the urine received in the bowl is very short, since the weight of the urine is only detected by the load cell when the urine is present in the receptor (on the load cell). Therefor any delaying of the urine flow before it enters the receptor should be minimized. For this reason, providing the bowl with a smooth surface is very advantageous. In addition, the bowl surface may especially be very well cleanable. Applicable material for the bowl unit may be, e.g., a hard polymeric material, a stainless steel, and a ceramic. It is even more advantageous if the bowl unit resembles a normal toilet bowl unit and the bowl unit further comprises a toilet seat, therewith minimally affecting the atmosphere, and thus the results of the measurement. Most normal toilets comprise a ceramic bowl. Thus, to resemble normal toilets providing a ceramic bowl in the toilet arrangement is expected to be most beneficial. Hence, in a specific embodiment, the toilet arrangement comprises a bowl unit comprises a ceramic bowl and the toilet arrangement further comprises a seat arrangable on the bowl.

In another specific embodiment, the toilet arrangement further comprises a bowl that has a substantially circular cross-section. The flow (pattern) of the urine in the bowl is determined by the shape and the surface of the bowl. As well as a smooth surface, a circular cross-section will disturb the flow (pattern) of the urine only minimally. Moreover, a bowl having a smooth surface with a substantially circular cross-section will enable a minimized residence time of the urine in the bowl unit. Furthermore, slowly decreasing the radius of the circular cross-section in the direction of the bowl unit outlet may even further minimize the residence time in the bowl and thus minimize the disturbance of the urine flow in the bowl. Further, minimizing the disturbance especially is advantageous because it will positively affect the results of the measurement. Hence, a specific embodiment of the toilet arrangement comprises a bowl unit comprising a tapering part with a tapering part length (i.e. a length over which the bowl tapers), tapering in a direction from a seat part to the bowl unit outlet, wherein the tapering part has a circular cross-section over the entire tapering part length. The tapering length may e.g. be in the range of 5-50 cm, such as 5-20 cm.

Further, the bowl unit may be provided with a bowl perfuming utility to cover up unpleasant odors. However, for hygienic reasons as well as for comforting the patients and the person leading or assisting the measurement, flushing the toilet arrangement after use is at least possible. Especially, if more than one patient has to undergo the measurement shortly after each other it is very advantageous if the toilet arrangement is cleaned after each measurement using the flushing system provided in the toilet arrangement. Having a clean toilet arrangement positively affects most patients, therewith improving the atmosphere during the measurement, positively affecting the results of the measurement. It also will have a positive effect on the working conditions of a person leading or assisting the measurement. Hence in a specific embodiment the toilet arrangement comprises a flushing unit.

The flushing unit may in an embodiment be configured to (temporarily) contain a flushing fluid, especially water, to flush the system (after the measurement). Alternatively, the flushing unit may (only) include a valve, configured to provide water to the bowl when desired (for instance, the action of providing water may be triggered by the control unit). The flushing unit may be functionally connected to a water service pipe, and may thus in an embodiment not necessarily include a water reservoir (similar to a washing machine). Hence, the flushing unit is especially functionally connectable to a source of water, such especially a water service pipe, and may optionally include a water storage. Therefore, the flushing unit is especially configured to flush the bowl of the bowl unit, more especially either with water directly obtained from a water service pipe or with water obtained from a (intermediate) reservoir. When using a reservoir, the reservoir may be functionally coupled to a water service pipe, similar as a conventional toilet may be.

Flushing may be provided to the bowl unit, so all parts downstream of the bowl unit may be cleaned by the action of the flushing fluid. Additionally, flushing may be provided to other parts of the toilet arrangement. Especially, it may be necessary to provide a flushing action directly to the receptor. Moreover, the flushing unit may be configured to simultaneously flush at different locations in the toilet arrangement or it may be configured to sequentially flush at different locations in the toilet arrangement. Further, the flushing fluid may be held in a container comprised by the flushing unit or instead or additionally the flushing fluid may originate from an external system used to provide the flushing fluid.

It is most desirable to flush the toilet arrangement every time a measurement is finished. In an embodiment, therefore the flushing unit comprises one or more handle(s) or button(s) to operate the flushing system manually. Most patients will be used to flush a (normal) toilet after use. However, since the urine flow measurement normally is performed at locations away from patient's own environment, it is expected that some patients may not use the flushing unit. In a specific embodiment, therefore, flushing is automated and initiated by the control unit at the moment the measurement is finished and the patient has left the toilet arrangement. Hence, the toilet arrangement may also include a sensor configured to sense a human or a movement of a human. Based thereon, the control unit may control the flushing unit. Hence, in an embodiment the control unit is further configured to control the flushing unit.

The receptor is especially configured to hold the urine during the measurement. After the measurement, the content of the receptor is pumped out of the receptor. In a specific embodiment, the pump functions as a valve and the receptor does not loose any volume if the pump unit is not in operation. In another embodiment the receptor also is provided with a valve and the receptor can only be emptied when the valve is opened, either manually or automatically, for instance by the control unit. The receptor must be capable to hold all of the urine plus accidental faces, and toilet paper provided during one measurement. Hence, the receptor comprises a receiving volume of at least 2 liter, and preferably the volume comprises more than 3 liter. The valve may especially be configured to open and close the receptor outlet.

Since urine is corrosive fluid, the receptor is constructed of material preferably not affected by urine (and feces), such as a polymeric material or a stainless steel, like stainless steel grades 304 or 316. As polymeric material especially one or more materials may be selected from the group consisting of, e.g., PE (polyethylene), PP (polypropylene), PEN (polyethylene napthalate), PC (polycarbonate), polymethylacrylate (PMA), polymethylmethacrylate (PMMA) (Plexiglas or Perspex), cellulose acetate butyrate (CAB), silicone, polyvinylchloride (PVC), polyethylene terephthalate (PET), (PETG) (glycol modified polyethylene terephthalate), PDMS (polydimethylsiloxane), and COC (cyclo olefin copolymer).

The receptor may in an embodiment include a substantially closed container comprising two openings, the receptor inlet and the receptor outlet. The latter may temporarily be closed with a valve during the measurement (see amongst others also above).

The total load on the load cell is (amongst others) determined by the receptor and the pump unit. The load cell, thus minimally is loaded by the initial weight of the pump unit and the receptor. Whereas during operation the total load on the load cell is determined by the initial weight of the pump unit and the receptor plus the weight of material (such as urine, feces, toilet paper, and flushing fluid) in the pump unit and in the receptor. To measure the weight of the material, especially the weight of the urine, in the receptor with the desired sensitivity, the capacity of the load cell is selected to be at least 1.2 times the sum of (the initial weight of the pump unit and the receptor plus the weight of the material in the pump unit plus 1 kg), more preferably the capacity of the load cell is selected in the rang from 1.2 times the sum of (the initial weight of the pump unit and of the receptor plus the weight of the material in the pump unit plus 1 kg) to 2 times the sum of (the initial weight of the pump unit and of the receptor plus the weight of the material in the pump unit plus 1 kg).

The receptor is especially configured to hold the urine during micturition. After the measurement, especially when the micturition has stopped, the content of the receptor is pumped out of the receptor (and/or the bowl unit is flushed). The control unit is functionally coupled to the load cell and is configured to detect the signal of the load cell. The signal of the load cell will stay substantially steady over a predefined period when the micturition has finished, triggering the end of the measurement moment for the control unit. Optionally or additionally, the control unit may detect a signal that a patient has left the toilet arrangement. The last signal may be provided by a senor provided in the toilet arrangement, for instance a pressure sensor or an IR sensor. The signal also may be provided by a button to be pushed manually. Optionally or additionally, the control unit may be configured to wait a predetermined time after start of the measurement or after a last change in the weight detection, and then pump the content out of the receptor and/or flush the bowl unit.

Much information can be deduced from the signal of the load cell. First of all, the signal of the load cell is used to analyze the measuring data. Next, if the signal of the load stays substantially steady over a predefined period the micturition has finished. The end of the measurement moment may be used by the control unit to control further actions, like starting the pumping unit and/or starting the flushing unit. Especially, these actions should not start immediately after the end of measurement moment. An extra period of time is required to enable patients to leave the toilet arrangement.

If the signal of the load cell suddenly shows a large increase it may, e.g., indicate that feces is received in the receptor. Because of the feces being excreted during the measurement, the measurement can not be used for a useful analysis and the measurement has to be repeated at another moment (or the feces contribution should be filtered out the signal). Further, knowing that feces are present in the system may also be advantageous for setting a more severe and controlled flushing action. Hence, in a specific embodiment, the toilet arrangement, further, comprises a control unit that is configured to detect from the load cell signal a feces contribution (or contribution of another material other than urine). In another specific embodiment of the toilet arrangement, the control unit may further be configured to filter (with software) the load cell signal for a feces contribution (or contribution of another material other than urine).

The control unit may further be provided with software for further processing of the load cell signal, such as software to remove the noise in the measurement, software to convert the measured weight over time into flow values, software to determine the moving average to filter outliers in the measured values, software to compare the measurement with previous data, etc., etc.. Further, all different kind of functions may be implemented in the control unit, controlling an output based on the (changes in) signal of the load cell. In an embodiment a signaling procedure is integrated in the control unit enabling to send a signal and inform another person, such as a nurse or experimental assistant, that the measurement is finished. The same signal or an analysis of the load cell signal may be used to trigger the pump unit to start emptying the receptor (after an extra period allowing the patient to leaf the toilet arrangement). At the same time or before starting the pump unit, the control unit may control the flushing unit. Hence, in a specific embodiment, wherein the toilet arrangement also comprising a flushing unit, the control unit is configured to control the flushing unit and the pump unit. Further, the control unit may include a transmitter configured to transmit (optionally wireless) the signal to a remote receiver, such as a computer or computer system of a hospital. In this way, a physician at a remote place may substantially directly analyze the urodynamic data.

The pump unit comprises a pump unit outlet. The pump unit outlet is provided to lead the waste of the toilet arrangement out of and away from the toilet arrangement. The waste of the toilet arrangement may comprises one or more of urine, (shredded) feces, (shredded) toilet paper, and flushing fluid.

In an embodiment, the waste is lead directly to a toilet drain, the same way a normal toilet discharges its waste to the sewer. However, this may require severe installation, breaking up floors, etc., especially since urinary flow measurements may be performed at different kind of locations, including locations having ample excess to toilet drains in close proximity. Therefore, it is advantageous to provide a system enabling the use of, e.g., a septic tank, a waste container, or a sink (with only small diamter exhaust tubes) or, especially the exhaust tubes of a sink for discharging the waste. Hence, in a specific embidiment, the toilet arrangement, further, comprises a pump unit outlet configured to be connected to a water exhaust tube (herein also indicated as "sink exhaust tube" or sink drain) having for instance a diameter selected from the range of 2-6 cm. Due to the shredding, it is (thus) not necessary to connect the toilet arrangement to a conventional toilet drain.

Since the urine flow measurements are especially performed at different kind of locations, such as in a (small) room, at a hospital laboratory, in a special prepared room resembling a toilet, etc., etc., the toilet arrangement, may especially be constructed as a stand alone unit that may be constructed easily at these different kinds of locations. Preferably, the location is provided with a water service to provide water for a flushing unit, and a means to which the toilet waste, hygienically, may be directed, such as a sink exhaust tube. It may even be more preferable that the toilet arrangement is removable and can be reconstructed easily at yet another location. Hence, in a specific embodiment the toilet arrangement is configured as single unit, optionally further comprising a flushing unit and a flushing unit connection for a connection with a water service pipe (at a location where the toilet unit is to be installed).

The toilet arrangement as described herein may be used for a urodynamic measurement of a human (during a defeacation activity). Such toilet arrangement may be installed for a predefined period at a predefined location, such as an elderly home, a doctor's practice, and a general practice center, a clinic, a hospital, etc., especially to (temporarily) perform urodynamic measurements of humans, especially of patients having urological problems, such as incontinence problems, obstruction, neurogenic bladder, etc., and for (then) for instance for determining and adjusting the diagnosis and the treatment plan. Especially, at these temporarily locations it is very advantageous if the toilet arrangement is configured as a single unit, further only requiring a water supply (tube) and a sink exhaust or even a container for the waste disposal. Hence, the construction of the toilet arrangement in existing buildings may be done very easily, without breaking walls, floors, etc.

Such a toilet arrangement also may be constructed (semi-) permanently in clinics or hospitals for performing recurring urodynamic measurements and incidental urodynamic measurements. The toilet arrangement, further, may especially be used by a pediatrician for a urodynamic measurement of a child, for instance having bedwetting problems.

In a further aspect, the invention provides a method of producing the toilet arrangement as described herein. Such method may especially comprise assembling the bowl unit and weight unit, and functionally connecting the control unit to the load cell. The method may further comprise selecting the bowl unit from a predefined set of bowl units, wherein predefined bowl units, for instance, may comprise one or more properties selected from the group comprising among others a ceramic bowl unit, a stainless steel bowl unit, a bowl having a tapered cross section, a bowl having an elliptic cross-section, a toilet seat, a lid, a bowl perfuming utility, an option to provide a connection with a flushing unit. In a specific embodiment also a flushing unit is provided to the toilet arrangement. The provided method results in a toilet arrangement described herein that may be constructed elsewhere and can be used for, especially, urodynamic measurements.

Therefore, in an embodiment software required to operate the toilet arrangement, especially for urodynamic measurements, such as software to detect the signal of the load cell required to control the pump unit and optionally the flushing unit, may be provided to the control unit during production of the toilet arrangement.

Optionally, the control unit, may be provided with software for analyzing the signal of the load cell with respect to the measurement and software for further processing of the load cell signal, such as software to remove the noise in the measurement, software to convert the measured weight over time into flow values, software to determine the moving average to filter outliers in the measured values, software to compare the measurement with previous data, etc..

In yet a further aspect, the invention provides a method of constructing the toilet arrangement described herein in an existing building, the method comprising providing the toilet arrangement described herein and functionally connecting the pump unit outlet with a water exhaust tube of said existing building. The method may further include construction such toilet arrangement including a flushing unit, and functionally connecting a flushing unit inlet to a water service pipe. Hence, the flushing unit may comprise a flushing unit connection for a connection with a water service pipe (from an existing building).

A main advantage of the toilet arrangement of the invention is the ease of construction. Construction does not require breaking of walls and/or floors associated with all kinds of long lasting, expensive operations. During operations (measurements) the pump unit will provide a flow of waste and optionally a flushing unit may require an inflow of flushing fluid. The waste flow coming from the pump unit outlet is shredded in the pump unit and easily pumpable through tubes having a diameter in the range of 2-6 cm. The waste flow may be directed to any kind of sanitary unit, such as a sink, or temporary waste storage container. Because of hygienic reasons, preferably the waste flow is directed to a water exhaust tube of the existing building. Therefore, in a specific embodiment, constructing the toilet arrangement comprises functionally connecting the pump unit outlet with a water exhaust tube of the building. Preferably the toilet arrangement also comprises a flushing unit. Especially, water should be provided to the flushing unit. Hence, in a specific embodiment, the method of constructing the toilet arrangement described herein comprises a toilet arrangement comprising a flushing unit described herein, the method further comprising functionally connecting the flushing unit to a water service pipe of said existing building.

Other main advantages are the ease of use and the much higher hygiene during use of the toilet arrangement, as well as more reliable measurements.

The terms "upstream" and "downstream" relate to an arrangement of items or features relative to the patch of the fluid flow, such as urine, excreted by the patient, wherein relative to a first position in the fluid flow, a second position in the path of the fluid flow closer to the point of excretion is "upstream", and a third position within path of the fluid flow further away from point of excretion is "downstream".

The term "substantially" herein, such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of". The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

The invention further applies to a device comprising one or more of the characterizing features described in the description and/or shown in the attached drawings. The invention further pertains to a method or process comprising one or more of the characterizing features described in the description and/or shown in the attached drawings.

The various aspects discussed in this patent can be combined in order to provide additional advantages. Furthermore, some of the features can form the basis for one or more divisional applications.

### BRIEF DESCRIPTION OF THE DRAWINGS

Embodiments of the invention will now be described, by way of example only, with reference to the accompanying schematic drawings in which corresponding reference symbols indicate corresponding parts, and in which:

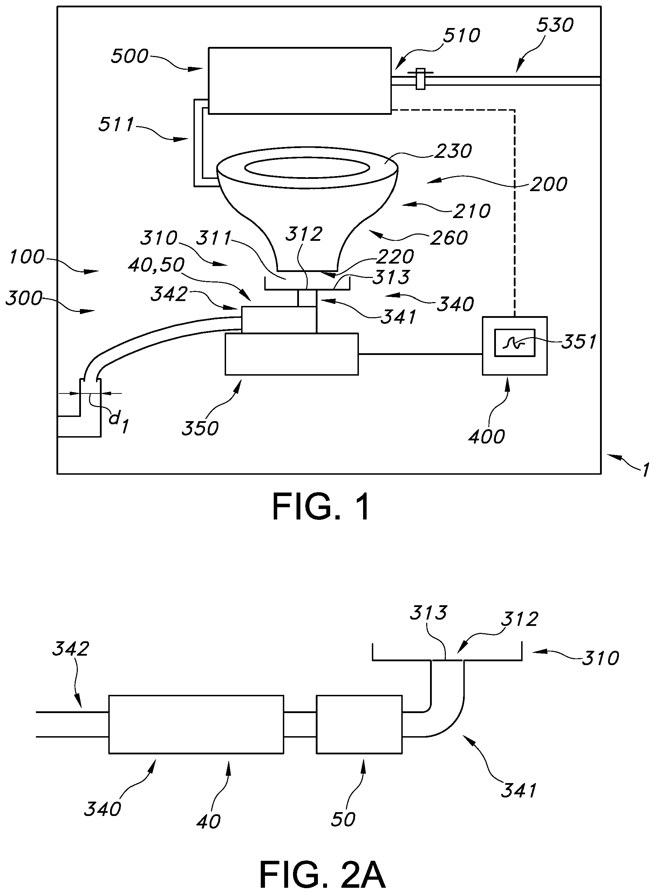

Figs. 1-3 schematically depict a number of embodiment and aspects of the invention

The drawings are not necessarily on scale.

### DETAILED DESCRIPTION OF THE EMBODIMENTS

Fig. 1 schematically depicts a building or room 1, such as a clinic, an elderly home, a doctor's practice, and a general practice center, a laboratory or observation room, etc. comprising a toilet arrangement 100.

The toilet arrangement 100 comprises a bowl unit 200, a weigh unit 300, a control unit 400 and, by way of example, in this embodiment also a flushing unit 500 is schematically depicted. Though the elements are indicated as separate parts, they may be integrated in a single unit. For instance, the flushing unit may include a magnet valve or other type of valve, which opens when necessary. The flushing unit may directly provide the flushing water to the bowl.

The bowl unit 200 comprises a bowl 210 and a bowl unit outlet 220 (here the outlet of the bowl 210). In operation, especially during a urine flow measurement, a human may take place on the bowl 210 and during micturition, urine flows down the bowl through the bowl unit outlet 220. The bowl unit 200 of the given embodiment, further, comprises a bowl seat 230, and a bowl 210 comprising a tapering part 260 supporting the direction of the urine flow. The urine flows out of the bowl unit 200 trough the bowl unit outlet 220 via the receptor opening 311 into the receptor 310 comprised by the weigh unit 300. Although the receptor opening 311 is in fluid contact with the bowl unit outlet 220, they are not in direct physical contact, so mass in the bowl unit is not weighed by the weigh unit 300.

The receptor 310, further comprises a receptor outlet 312. Next to the receptor 310, the weigh unit 300 comprises a pump unit 340 and a load cell 350. The pump unit inlet 341 comprised by the pump unit 340 is in fluid contact with the receptor outlet 312. The pump unit 340, therefor may function as a valve for outflow of material present in the receptor when the pump unit is not in operation or the pump unit may comprise a valve.

Additionally the receptor 310 may optionally comprise a valve 313. The pump unit 340 further comprises a shredder 50 in fluid contact with the receptor outlet 313, a pump 40, and a pump unit outlet 342. The shredder is especially comprised in case faces, toilet paper or other (semi-) solids are provided in the pump unit 340. The shredder 50 may be located before (upstream of) the pump 40 or after (downstream of) the pump 40. Moreover the shredder 50 and pump 40 also may be combined in one apparatus in the pump unit 340.

The load cell 350 is especially configured to weigh the urine in the receptor 310. Therefore it is configured to weigh the total of the weigh unit 300 before the measurement and during the measurement when urine enters the receptor 310. The load cell 350 provides the corresponding signal to the control unit 400. Waste from the pump unit, such as urine, feces, toilet paper, and flushing liquid is discharged in a water exhaust tube with a diameter d1 which may be between 2 and 6 cm. The flushing unit 500 is functionally connected to a conventional water infrastructure 530 via a flushing unit connection line 510. Connection between the flushing unit and the tubing bowl unit and/or the weigh unit (not pictured) is provided by tubing 511.

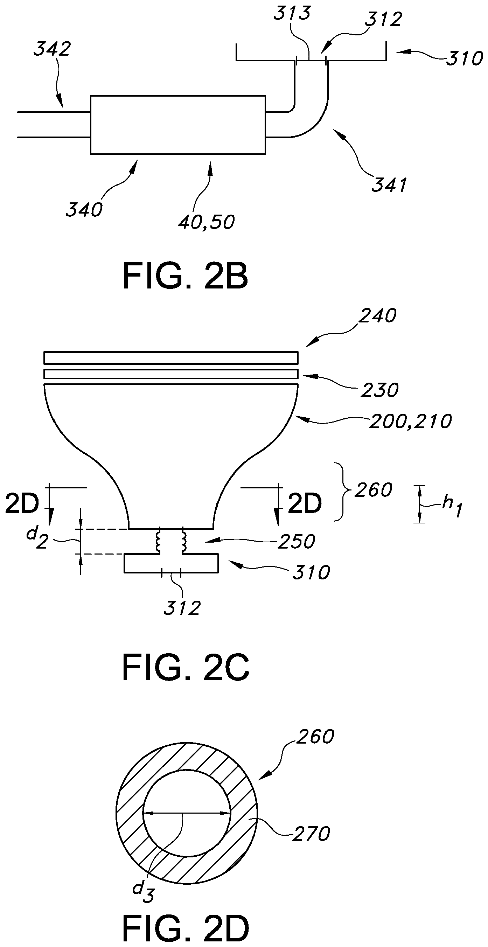

Figs 2a-2b schematically depict some aspects of the receptor 310 and downstream of the receptor 310. In the embodiment given in Fig 2a, the receptor 310 comprises a physical valve 313 closing the receptor opening 312, and a shredder 50 is provided upstream of the pump 40. In the embodiment given in Fig 2b, the pump unit 340 functions as a valve or comprises a valve and the receptor opening 312 is open as indicated by a not completely closed valve 313. Further, the pump 40 and shredder 50 are combined in one apparatus.

Figure 2c schematically depicts an embodiment of the bowl unit 200 comprising a bowl 210. The bowl 210 comprises a bowl seat 230 and a bowl closure 240 that can be closed after the experiment. The bowl comprises a substantially circular cross-section (see Fig 2d for the cross-section of Fig 2c) and comprises a tapering part 260 with a tapering part length h1, tapering in a direction from the seat part to the receptor 310. In this embodiment, a flexible connection is provided between the bowl unit outlet 220 and the receptor 310. Because the weight of the bowl unit 200 should not be measured by the load cell 350, only a flexible connection 250 may be provided between the bowl unit outlet 220 and the receptor 310. The length d2, however should be minimized to prevent material to be left in the flexible connection 250.

Fig. 2d schematically depicts a cross-section of the bowl 210 given in Fig 2c at the location 2D, and shows the substantially circular cross-section with a diameter d3 of the tapering part 260. Note that the diameter d3 decreases with an increased tapering. The bowl wall 270, preferably comprises ceramic.

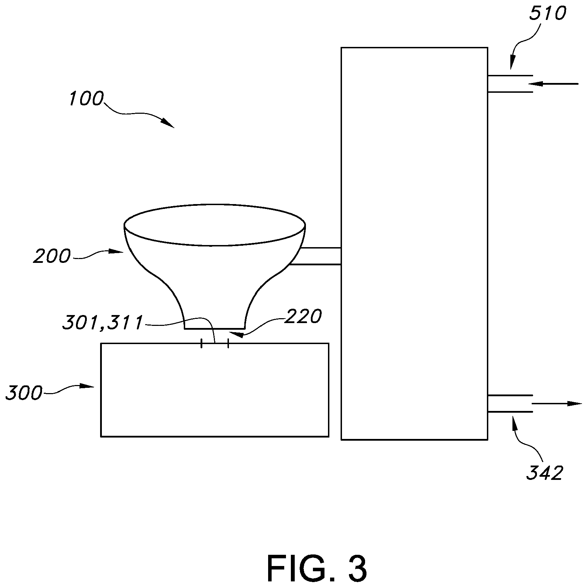

Fig. 3 schematically depicts a toilet arrangement 100, comprising a bowl unit 200 and a weigh unit 300, constructed in a room or building 1. The receptor opening 311 may comprise different shaped openings 301. In the present embodiment a circular opening 301 is comprised whereas in Figs. 2a and 2b larger rectangular openings are comprised. The construction of the toilet arrangement 100 comprises a flushing unit connected to a conventional water infrastructure 530 by a flushing connection unit 510. The waste is discharged from the toilet arrangement via the pump unit outlet 342 to, e.g., a water exhaust tube.
- d1: diameter water exhaust tube
- d2: distance
- d3: diameter of tapering part
- h1: tapering part length
- 1: building/room
- 40: pump
- 50: shredder
- 100: toilet arrangement
- 200: bowl unit
- 210: bowl
- 220: bowl unit outlet
- 230: bowl seat
- 240: bowl closure
- 250: flexible connection
- 260: tapering part
- 300: weigh unit
- 301: opening
- 310: receptor
- 311: receptor opening
- 312: receptor outlet
- 313: valve
- 340: pump unit
- 341: pump unit inlet
- 342: pump unit outlet
- 350: load cell
- 400: control unit
- 500: flushing unit
- 510: flushing unit connection line
- 511: tubing
- 530: conventional water infrastructure

## Claims

1. A toilet arrangement for a urodynamic measurement, the toilet arrangement comprising:
- a bowl unit with a bowl unit outlet;
- a weigh unit comprising: (i) a receptor comprising (a) a receptor opening in fluid contact with the bowl unit outlet but not in direct physical contact with the bowl unit outlet and (b) a receptor outlet; (ii) a pump unit comprising (a) a pump unit inlet in fluid contact with the receptor outlet, (b) a shredder in fluid contact with the receptor outlet, and (c) a pump unit outlet; (iii) a load cell configured to weigh the receptor and the pump unit and to provide a corresponding load cell signal; and
- a control unit configured to detect the load cell signal as function of time.

2. The toilet arrangement according to claim 1, wherein the pump unit comprises a pump with an integrated shredder.

3. The toilet arrangement according to any one of the preceding claims, further comprising a flushing unit.

4. The toilet arrangement according to claim 3, wherein the control unit is configured to control the flushing unit and the pump unit.

5. The toilet arrangement according to any one of the preceding claims, wherein the pump unit outlet is configured to be connected to a water exhaust tube having a diameter selected from the range of 2-6 cm.

6. The toilet arrangement according to any one of the preceding claims, wherein the control unit is configured to detect from the load cell signal a feces contribution.

7. The toilet arrangement according to any one of the preceding claims, wherein the control unit is configured to filter the load cell signal for a feces contribution.

8. The toilet arrangement according to any one of the preceding claims, wherein the bowl unit comprises a ceramic bowl and wherein the toilet arrangement further comprises a seat arrangable on the bowl.

9. The toilet arrangement according to any one of the preceding claims, wherein the bowl has a substantially circular cross-section.

10. The toilet arrangement according to any one of the preceding claims, wherein the bowl comprises a tapering part with a tapering part length, tapering in a direction from a seat part to the bowl unit outlet, wherein the tapering part has a circular cross-section over the entire tapering part length.

11. The toilet arrangement according to any one of the preceding claims, configured as single unit and further comprising the flushing unit according to any one of claims 3-4 and a flushing unit connection for a connection with a water service pipe.

12. Use of the toilet arrangement according to any one of claims 1-11 for a urodynamic measurement of a human.

13. A method of producing the toilet arrangement according to any one of claims 1-11, the method comprising assembling the bowl unit and weight unit, and functionally connecting the control unit to the load cell.

14. A method of constructing the toilet arrangement according to any one of claims 1-11 in an existing building, the method comprising providing the toilet arrangement according to any one of claims 1-11 and functionally connecting the pump unit outlet with a water exhaust tube of said existing building.

15. The method according to claim 14, wherein the toilet arrangement further comprises the flushing unit according to any one of claims 3, 4 and 11, the method further comprising functionally connecting the flushing unit to a water service pipe of said existing building.

## Patentansprüche

1. Toilettenanordnung für eine urodynamische Messung, wobei die Toilettenanordnung umfasst:
eine Schüsseleinheit mit einem Schüsseleinheitauslass;
eine Wiegeeinheit, umfassend: (i) einen Empfänger umfassend (a) eine Empfängeröffnung in Fluidkontakt mit dem Schüsseleinheitauslass, jedoch nicht in direktem physischen Kontakt mit dem Schüsseleinheitauslass, und (b) einen Empfängerauslass; (ii) eine Pumpeneinheit umfassend (a) einen Pumpeneinheiteinlass in Fluidkontakt mit dem Empfängerauslass, (b) einen Zerkleinerer in Fluidkontakt mit dem Empfängerauslass und (c) einen Pumpeneinheitauslass; (iii) eine Wägezelle, die so ausgelegt ist, dem Empfänger und die Pumpeneinheit zu wiegen und ein entsprechendes Wägezellensignal bereitzustellen; und
eine Steuereinheit, die so ausgelegt ist, das Wägezellensignal als Funktion der Zeit zu erkennen.

2. Toilettenanordnung nach Anspruch 1, wobei die Pumpeneinheit eine Pumpe mit einem integrierten Zerkleinerer umfasst.

3. Toilettenanordnung nach einem der vorstehenden Ansprüche, weiterhin umfassend eine Spüleinheit.

4. Toilettenanordnung nach Anspruch 3, wobei die Steuereinheit dazu ausgelegt ist, die Spüleinheit und die Pumpeneinheit zu steuern.

5. Toilettenanordnung nach einem der vorstehenden Ansprüche, wobei der Pumpeneinheitauslass dazu ausgelegt ist, mit einer Wasserablassrohrleitung mit einem Durchmesser ausgewählt aus dem Bereich von 2-6 cm verbunden zu werden.

6. Toilettenanordnung nach einem der vorstehenden Ansprüche, wobei die Steuereinheit dazu ausgelegt ist, aus dem Wägezellensignal einen Fäkalienbeitrag zu erkennen.

7. Toilettenanordnung nach einem der vorstehenden Ansprüche, wobei die Steuereinheit dazu ausgelegt ist, das Wägezellensignal nach einem Fäkalienbeitrag zu filtern.

8. Toilettenanordnung nach einem der vorstehenden Ansprüche, wobei die Schüsseleinheit eine Keramikschüssel umfasst und wobei die Schüsseleinheit weiterhin einen auf der Schüssel anordbaren Sitz umfasst.

9. Toilettenanordnung nach einem der vorstehenden Ansprüche, wobei die Schüssel einen im Wesentlichen kreisförmigen Querschnitt aufweist.

10. Toilettenanordnung nach einem der vorstehenden Ansprüche, wobei die Schüssel einen sich verjüngenden Teil mit einer sich verjüngenden Teilelänge umfasst, der sich in einer Richtung von einem Sitzteil zum Schüsseleinheitauslass verjüngt, wobei der sich verjüngende Teil über der gesamten sich verjüngenden Teilelänge einen kreisförmigen Querschnitt aufweist.

11. Toilettenanordnung nach einem der vorstehenden Ansprüche, ausgelegt als einzelne Einheit und weiterhin umfassend die Spüleinheit nach einem der Ansprüche 3-4 und eine Spüleinheitverbindung für eine Verbindung mit einem Wasserversorgungsrohr.

12. Verwendung der Toilettenanordnung nach einem der Ansprüche 1-11 für eine urodynamische Messung eines Menschen.

13. Verfahren zum Herstellen der Toilettenanordnung nach einem der Ansprüche 1-11, wobei das Verfahren das Montieren der Schüsseleinheit und Wiegeeinheit und das funktionsfähige Verbinden der Steuereinheit mit der Wägezelle umfasst.

14. Verfahren zum Konstruieren der Toilettenanordnung nach einem der Ansprüche 1-11 in einem bestehenden Gebäude, wobei das Verfahren weiterhin das Bereitstellen der Toilettenanordnung nach einem der Ansprüche 1-11 und das funktionsfähige Verbinden des Pumpeneinheitauslasses mit einer Wasserablassrohrleitung dieses bestehenden Gebäudes umfasst.

15. Verfahren nach Anspruch 14, wobei die Toilettenanordnung weiterhin die Spüleinheit nach einem der Ansprüche 3, 4 und 11 umfasst, wobei das Verfahren weiterhin das funktionsfähige Verbinden der Spüleinheit mit einem Wasserversorgungsrohr dieses bestehenden Gebäudes umfasst.

## Revendications

1. Agencement de toilette pour une mesure urodynamique, l'agencement de toilette comprenant :
- une unité faisant office de cuvette comprenant une sortie d'unité faisant office de cuvette ;
- une unité de pesée comprenant : (i) un récepteur comprenant (a) une ouverture de récepteur en contact par fluide avec la sortie de l'unité faisant office de cuvette, mais non en contact physique direct avec la sortie de l'unité faisant office de cuvette, et (b) une sortie de récepteur; (ii) une unité faisant office de de pompe comprenant (a) une entrée d'unité faisant office de pompe en contact par fluide avec la sortie du récepteur, (b) un broyeur en contact par fluide avec la sortie du récepteur, et (c) une sortie d'unité faisant office de pompe ; (iii) un dynamomètre configuré pour peser le récepteur et l'unité faisant office de pompe et pour procurer un signal de dynamomètre correspondant ; et
- une unité de commande configurée pour détecter le signal de dynamomètre en fonction du temps.

2. Agencement de toilette selon la revendication 1, dans lequel l'unité faisant office de pompe comprend une pompe équipée d'un broyeur intégré.

3. Agencement de toilette selon l'une quelconque des revendications précédentes, comprenant en outre une unité faisant office de chasse.

4. Agencement de toilette selon la revendication 3, dans lequel l'unité de commande est configurée pour commander l'unité faisant office de de chasse et l'unité faisant office de de pompe.

5. Agencement de toilette selon l'une quelconque des revendications précédentes, dans lequel la sortie de l'unité faisant office de pompe est configurée pour être reliée à un tube d'évacuation d'eau dont le diamètre est choisi dans la plage de 2 à 6 cm.

6. Agencement de toilette selon l'une quelconque des revendications précédentes, dans lequel l'unité de commande est configurée pour détecter, à partir du signal du dynamomètre, un apport de matières fécales.

7. Agencement de toilette selon l'une quelconque des revendications précédentes, dans lequel l'unité de commande est configurée pour filtrer le signal de dynamomètre pour un apport de matières fécales.

8. Agencement de toilette selon l'une quelconque des revendications précédentes, dans lequel l'unité faisant office de cuvette comprend une cuvette en céramique et dans lequel l'agencement de toilette comprend en outre un siège qui peut venir se disposer sur la cuvette.

9. Agencement de toilette selon l'une quelconque des revendications précédentes, dans lequel la cuvette possède une section transversale essentiellement circulaire.

10. Agencement de toilette selon l'une quelconque des revendications précédentes, dans lequel la cuvette comprend une partie tronconique avec une longueur de partie tronconique, qui se rétrécit dans la direction allant d'une partie faisant office de siège jusqu'à la sortie de l'unité faisant office de cuvette, la partie tronconique possédant une section transversale circulaire sur toute la longueur de la partie tronconique.

11. Agencement de toilette selon l'une quelconque des revendications précédentes, configuré sous la forme d'une seule pièce et comprenant en outre l'unité faisant office de chasse selon l'une quelconque des revendications 3 à 4 et un raccord d'unité faisant office de chasse pour une liaison à un tuyau de branchement d'eau.

12. Agencement de toilette selon l'une quelconque des revendications 1 à 11, pour une mesure urodynamique d'un être humain.

13. Procédé de fabrication de l'agencement de toilette selon l'une quelconque des revendications 1 à 11, le procédé comprenant le montage de l'unité faisant office de cuvette et de l'unité de pesée, et la liaison fonctionnelle de l'unité de commande au dynamomètre.

14. Procédé de construction de l'agencement de toilette selon l'une quelconque des revendications 1 à 11 dans un bâtiment existant, le procédé comprenant le fait de procurer l'agencement de toilette selon l'une quelconque des revendications 1 à 11 et d'établir une liaison fonctionnelle entre la sortie de l'unité faisant office de pompe et un tube d'évacuation d'eau dudit bâtiment existant.

15. Procédé selon la revendication 14, dans lequel l'agencement de toilette comprend en outre l'unité faisant office de chasse selon l'une quelconque des revendications 3, 4 et 11, le procédé comprenant en outre le fait d'établir une liaison fonctionnelle entre l'unité faisant office de chasse et un tube d'évacuation d'eau dudit bâtiment existant.
